# EUROPEAN PATENT APPLICATION

(11) **EP 2 216 402 A1**
(43) Date of publication of application: **11.08.2010**
(21) Application number: 08805392.1
(22) Date of filing: 24.07.2008
(51) Int. Cl.: C12N 9/64, C12N 15/69, C12N 15/81

(54) **USE OF ACTIVE RECOMBINANT CHYMOSIN**

(30) Priority: 27.07.2007 ES 200702111
(71) Applicant: Universidade De Santiago De Compostela, 15782 Santiago de Compostela (ES)
(72) Inventor: GONZALEZ VILLA, Tomás, E-15782 Santiago De Compostela (ES); VALLEJO VIDAL, Juan Andrés, E-15782 Santiago De Compostela (ES); POZA DOMÍNGUEZ, Margarita, E-15782 Santiago De Compostela (ES); AGEITOS MARTÍNEZ, José Manuel, E-15782 Santiago De Compostela (ES)
(74) Representative: Pons Ariño, Angel
(86) International application number: PCT/ES2008/070147
(87) International publication number: WO 2009/027572

(57) **Abstract**

Use of rennet, without prior activation, obtained from a host cell that contains an expression vector that comprises any of the sequences coding for the buffalo proteins selected from the group: preprochymosin, prochymosin and chymosin for the coagulation of milk.

## Description

### USE OF ACTIVE RECOMBINANT CHYMOSIN

The present invention refers to the use of recombinant buffalo chymosin for making cheese. This chymosin is produced in its active form by direct secretion by the host microorganism, without the need of an intermediary autocatalytic activation process mediated by pH.

### STATE OF PRIOR ART

Rennets used in the cheese industry are basically composed of aspartic proteases (Ref. Ustunol & Hicks., 1990, Journal of Dairy Science, vol. 73(1), pp. 8-16). Aspartic proteases have two residues of aspartic acid that are essential for their operation: Asp32 and Asp215. This feature, together with its two-lobed three-dimensional confirmation, where the active centre rich in aspartic acid is located between both lobes, gives them the capacity to act on the Phe105-Met106 peptide bond of milk κ-casein, giving insoluble para-κ-casein (Ref. McMahon et al., 1984, Journal of Dairy Science, vol. 67(5), pp. 919-29). This leads to coagulation of the milk, explaining why these proteases have been used for the traditional preparation of cheese masses as a prior step in the manufacture of various types of cheese.

Chymosin is the most valued aspartic protease in the cheese industry as a source of rennet for its effectiveness in making cheese masses (Ref. Rao et al., 1998, Microbiology and Molecular Biology Reviews, vol. 62(3), pp. 597-635). Other aspartic proteases used in the cheese industry give non-specific digestions of milk caseins and can give rise to products giving a bitter taste to the cheese masses and to losses of protein content during the draining process, which causes a significant reduction in yield in the process of making the cheese (Ref. Beppu., 1983, Trends in Biotechnology, vol. 1 (3), pp. 85-9).

Chymosin is synthesised *in vivo* in the form of preprochymosin containing a 16-amino acid signal peptide, which is removed to give prochymosin. Prochymosin in turn is processed to active chymosin by being subjected to acid pH conditions in the stomach that causes the removal of 42 amino acids located at the extreme amino terminal of the propeptide (Ref. V B Pedersen et al., 1979, European Journal of Biochemistry, vol. 94(2), pp. 573-80).

Currently there are four sources of various origins for the production of rennet for the cheese industry: animals, plants, microbes and recombinants. Rennet of animal origin (chymosin) has traditionally been obtained starting from the direct pressing of the rennet-bag of unweaned calves. This rennet of animal origin is not capable of meeting the current market demand. Additionally, its use brings with it the risk of appearance of impurities due to the method of extraction and also a risk of incorporating infectious agents (e.g. Creutzfeld-Jacob).

Rennets of plant origin have been obtained starting from infusions of flowers such as *Cynara cardunculus* (Ref. Cordeiro et al., 1994, Plant Molecular Biology, vol. 24(5), pp. 733-41) or *Gallum verum.* These rennets are incapable of competing industrially so they have been relegated to making traditional cheeses and their use has been reduced to specific geographic areas.

Microbial rennets are mainly obtained from the supernatants of microorganism cultures such as *Endothia* (Ref. Droehse, Helle B et al., Biochimica et Biophysica Acta, Protein Structure and Molecular Enzymology, vol. 995(3), pp 221-4) or *Rhizomucor* (Ref. Sternberg, 1972, Biochimica et Biophysica Acta, Protein Structure, vol. 285(2), pp. 383-92). These rennets contain poorly specific proteases that cause bitter flavours and a reduction in the yield during the process of making cheese masses.

Lastly, commercialised recombinant rennets have so far been obtained from recombinant microorganisms containing the chymosin gene of *Bos taurus* (Ref. Emtage et al., 1983, Proceedings of the National Academy of Sciences of the United States of America, vol. 80(12), pp. 3671-5; Mellor et al., 1983, Gene, vol. 24(1), pp 1- 14; Cullen et al., 1987, Bio/Technology, vol. 5(4), pp. 369-76). This recombinant chymosin behaves in the same way as that obtained from direct pressing of rennet-bags with the advantage that its use now avoids the appearance of impurities and infectious agents.

These types of recombinant chymosins must be activated by an autocatalytic process mediated by a simulation of the acid pH of the animal stomach. Only then can they be used for the making of cheese (Ref. V B Pedersen et al., 1979, European journal of biochemistry, vol. 94(2), pp. 573-80).

### DESCRIPTION OF THE INVENTION

The present invention refers to obtaining active recombinant buffalo chymosin capable of coagulating milk from different sources for making cheese masses. This buffalo chymosin, after cloning in the yeast *Pichia pastoris* is secreted by the microorganism into the extracellular medium in its active form. This is, without the need to be activated later by simulation of the pH of the stomach. This represents an optimisation in the process of making cheeses, with a saving of time and money in obtaining the product, considerably increasing the yield in its production.

Therefore, the use of recombinant buffalo chymosin, which is not subjected to the process of activation by pH, represents an additional advantage compared to the state of the art, avoiding an intermediate activation process for the making of cheese masses.

Buffalo chymosin shows more stable physico-chemical properties than that from *Bos taurus* (Ref. Mohanty et al.; 2003, Journal of Dairy Research, vol. 70(1), pp 37-43; Shindo & Arima; 1979, Rakuno Kagaku, Shokuhin no Kenkyu, vol. 28(5), A177-A182) and it has been shown that using buffalo chymosin gives a higher yield in the making of cheese masses using buffalo milk. This is useful for the production of cheeses and in particular of mozzarella, a characteristic cheese originating from buffalo milk.

Another factor to take into account is that in the sequence of the chymosin gene from *Bos taurus,* there is an EcoRl target that does not exist in the sequence of *Bubalus arnee bubalis.* This difference can be used, for example, for differentiating a recombinant strain producing chymosin from *Bos taurus* from another strain producing chymosin from *Bubalus arnee bubalis.*

Thus, an aspect of the present invention refers to the use of the rennet obtained directly from a host cell in which the gene has been cloned in an expression vector, for the coagulation of milk. In this vector, the three forms of the gene have been cloned, coding for the buffalo proteins selected from the sequences of the preprochymosin, the prochymosin and the chymosin. The rennet thus obtained does not require prior activation to carry out the coagulation of milk.

The term "milk" is understood in the present invention to be the product obtained from the females of mammals, in particular of domestic mammals such as cows, goats, sheep, horses, camels, buffalos, etc. The coagulation can be carried out in the types of milk described above or in any of their mixtures.

The term "rennet" is understood to be the product obtained by extraction from ruminant rennet-bags, in the present invention buffalo rennet, which serves to coagulate milk. This rennet is the supernatant obtained on cloning buffalo chymosin in a microorganism and secreted into the extracellular medium, and comprises an enzyme, active recombinant buffalo chymosin.

The term "active recombinant chymosin" in this description means the recombinant chymosin secreted by the host organism directly in its active form, that is, without the need to carry out an intermediate auto-catalytic process, mediated by pH, before its use in the making of cheese. This active recombinant chymosin is preferably from buffalo.

The host microorganism can be, but without limitation, yeasts of the phylum *Ascomycota* such as *Saccharomyces, Schizosaccharomyces, Kluyveromyces* and *Pichia.* In a preferred embodiment of the present invention, the microorganism is *Pichia pastoris.*

Another preferred embodiment of the present invention comprises the use of the active recombinant buffalo rennet for the making of cheese, where the cheese can be obtained from any type of milk or mixtures, more preferably for the making of buffalo cheese, called mozzarella.

The rennet, which contains the chymosin enzyme, preferably from recombinant *Pichia pastoris* with the gene for buffalo chymosin, is capable of making cheese masses more quickly than the recombinant rennets known in the state of the art and does not need any prior process of activation, in contrast to the recombinant rennets currently known.

From various combinations of oligonucleotides designed both from bovine preprochymosin and from buffalo prochymosin, the sequences of buffalo preprochymosin, prochymosin and chymosin are obtained. Obtaining these DNA sequences can be achieved by RT-PCR.

An expression vector is constructed that contains the three DNA sequences obtained, in order to be later used for the expression of these sequences in a host microorganism, which is preferably *Pichia pastoris.*

Throughout the description and the claims, the word "comprise" and its variants is not intended to exclude other technical characteristics, additives, components or steps. For experts in the field, other objects, advantages and characteristics of the invention will become apparent from the description and from the practice of the invention. The following examples and figures are provided by way of illustration and are not intended to be limiting of the present invention.

### BRIEF DESCRIPTION OF THE FIGURES

Fig. 1. Shows oligonucleotides designed from the sequence of *Bos taurus* preprochymosin (NM_180994) and the sequence of *Bubalus arnee bubalis* prochymosin (AF_177290).
Fig. 2. Shows the pCR Blunt II TOPO + Preprochymosin vector.
Fig. 3. Shows the pCR Blunt II TOPO + Prochymosin vector.
Fig. 4. Shows the pCR Blunt II TOPO + Chymosin vector.
Fig. 5. Shows the pGAPZαA expression vector.
Fig. 6. Shows the coagulation trials of recombinant *Pichia pastoris* colonies.
Fig. 7. Shows cheese masses made with cow milk and rennet from recombinant *Pichia pastoris* with the buffalo chymosin gene.

### DETAILED DESCRIPTION OF METHODS OF EMBODIMENT

The invention is illustrated below by means of trials carried out by the inventors, which show the specificity and effectiveness of the use of active recombinant buffalo chymosin.

In the following examples, it is shown how RNA is extracted starting from the abomasum of an infant water buffalo (*Bubalus arnee bubalis*). Using various combinations of oligonucleotides designed both from *Bos taurus* preprochymosin and from *Bubalus arnee bubalis* prochymosin, the sequences of buffalo preprochymosin, prochymosin and chymosin were obtained by RT-PCR. These showed a degree of homology with the equivalent bovine proteins of 97.9, 97.1 and 96.8%, respectively. The three DNA sequences obtained were cloned into the pCR-Blunt II-TOPO vector to be later transferred to the pGAPZαA vector. Using the construction obtained from cloning in the integrative vector pGAPZαA, three recombinant clones of *Pichia pastoris* were obtained. The three recombinant *Pichia pastoris* clones contained integrated in their genome the sequences corresponding to *Bubalus arnee bubalis* preprochymosin, prochymosin and chymosin. These recombinant clones were cultivated in YPD liquid medium without selective pressure and the culture supernatants contained the milk coagulation activity. The yeast *Pichia pastoris* efficiently expressed the three protein forms corresponding to *Bubalus arnee bubalis* preprochymosin, prochymosin and chymosin and these also appeared in an already active state in the culture supernatant. The supernatant obtained from recombinant *Pichia pastoris* was subjected to coagulation assays and it was found that in three cases that rennet thus obtained was capable of coagulating various types of milk, making cheese masses of high quality in a short time.

### EXAMPLE

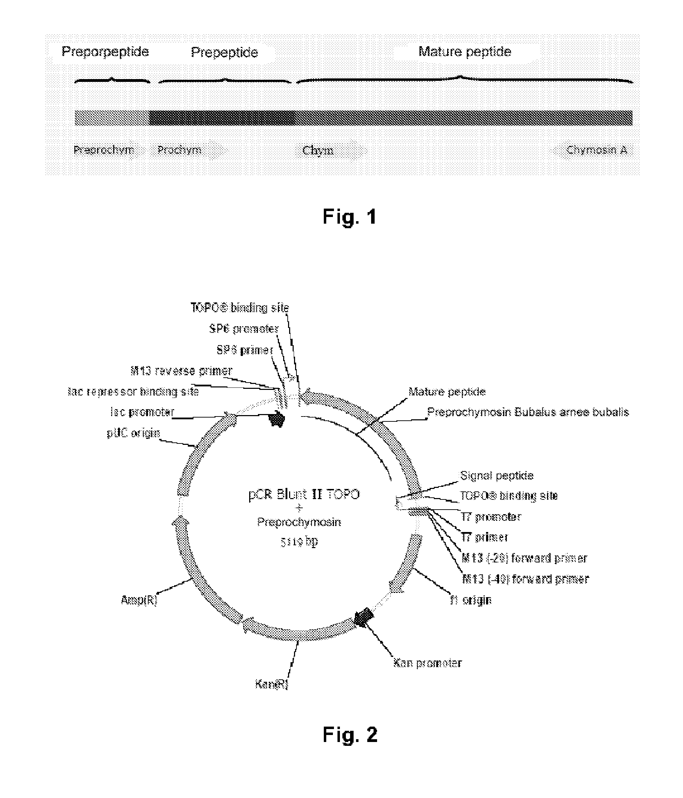

The rennet-bag of an individual male infant *Bubalus arnee bubalis* was extracted. The tissue was chopped and submerged in a RNA stabiliser; "RNAlater Stabilization Reagent" (Qiagen). To each 30 mg sample of tissue, 300 µl of reagent were added. This treatment prevents degradation of the RNA in the tissue. Then, the tissue was frozen using liquid nitrogen and was kept at - 80 ºC for an indefinite period. The RNA was isolated from the rennet-bag using the RNeasy Plant Mini Kit (Qiagen) and following the instruction recommended by the manufacturer. After extracting the RNA, RT-PCR trials were performed to obtain cDNA molecules corresponding to preprochymosin, prochymosin and chymosin. To perform these RT-PCR trails, three pairs of oligonucleotides or primers (Fig. 1) were designed using the sequence of *Bos taurus* preprochymosin (NM_180994) and the sequence of *Bubalus arnee bubalis* prochymosin (AF_177290).
Preprochym: 5'- ATG AGG TGT CTC GTG GTG CTA CTT- 3' (SEQ NO: 1) Prochym: 5'- ATGGCAGAAATCACCAGAATCCCTC- 3' (SEQ NO: 2)
Chym: 5'- ATGGGTGAGGTAGCGAGCGTGCC- 3' (SEQ NO: 3)
Chymosin A: 5'- TCAGATAGCCTTCGCCAGCCC -3' (SEQ NO: 4)

The first strand of cDNA was synthesised at 42 ºC for one hour performing various reactions using different combinations of the oligonucleotides; Oligo-p(dT)15, Random p(dN)6 and Chymosin A. The enzyme used for the synthesis of the first strand was AMV reverse transcriptase (Roche).

Once the first strand had been obtained, the cDNA was obtained using the three possible pairs of primers previously described by means of PCR. The reaction mixture contained: polymerase buffer 1X, 0.2 mM of each oligonucleotide, 1.5 nM of MgCl2, 0.2 mM of a mixture of the four deoxynucleotides, 1 U of Accuzyme DNA Polymerase (Bioline), as template the product of reaction of the synthesis of the first strand and the mixture was made up with water to 50 microlitres. This polymerase leaves blunt ends that are necessary for the subsequent introduction of the PCR product into the cloning vector pCR Blunt II TOPO (Invitrogen). The PCR reactions were carried out in a Robocycler temperature cycler 96 (Stratagene) following a programme of 96 °-C for 2 min, 35 cycles of 96 ºC for 1 min, 57 ºC for 1 min and 72 ºC for 2 min and a final incubation at 72 ºC for 5 min.

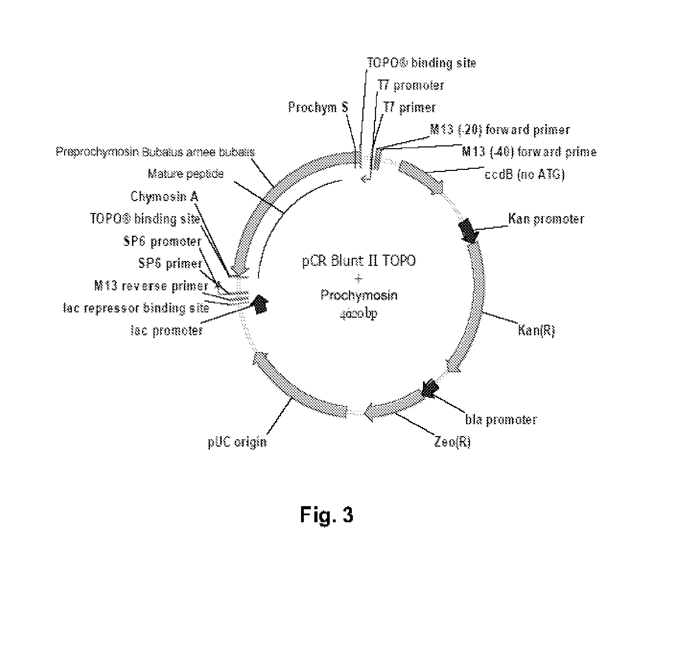

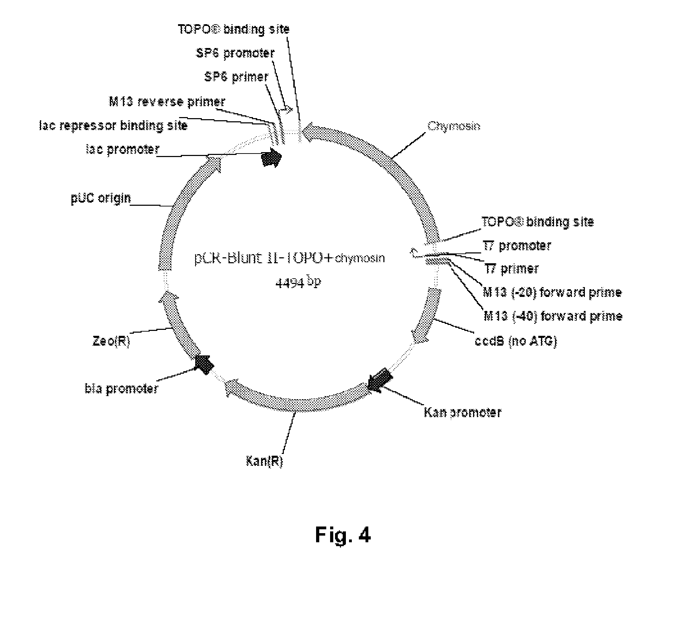

The three PCR products obtained were cloned into the pCR BluntII TOPO (Invitrogen) vector. The cloned fragments were sequenced. A homology of around 97% was found between the sequences of *Bos taurus* and of *Bubalus arnee bubalis* and a different EcoRI digestion pattern was found between the two sequences. Thus, three different constructions in the pCR Blunt II TOPO vector containing respectively the sequences of buffalo preprochymosin, prochymosin and chymosin (Figs. 2, 3, and 4).

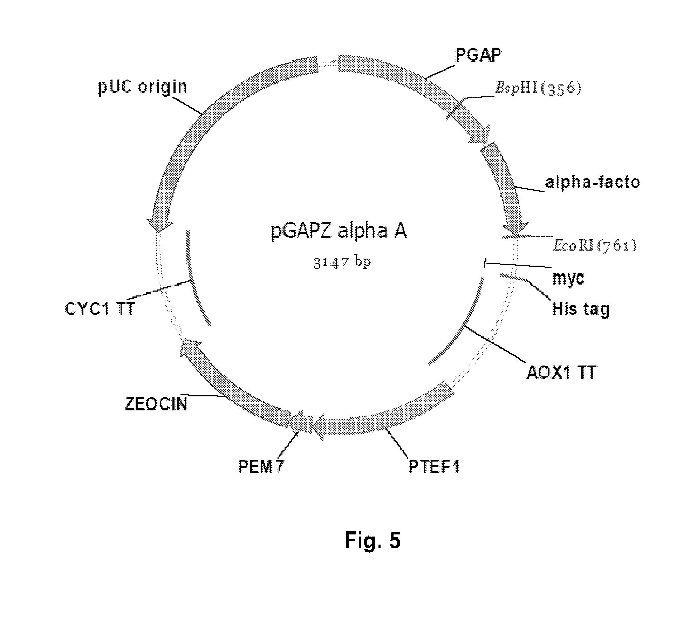

For the expression of the three sequences corresponding to preprochymosin, prochymosin and chymosin in *Pichia pastoris,* the pGAPZαA integrative vector was used, enabling the extracellular secretion of the gene after cloning in the reading frame with the signal peptide sequence of factor α of *Saccharomyces cerevisiae* and under the control of the constitutive promoter pGAP. This vector also contains the terminator of the AOX gene that provides the yeast with resistance to zeocine (Fig. 5).

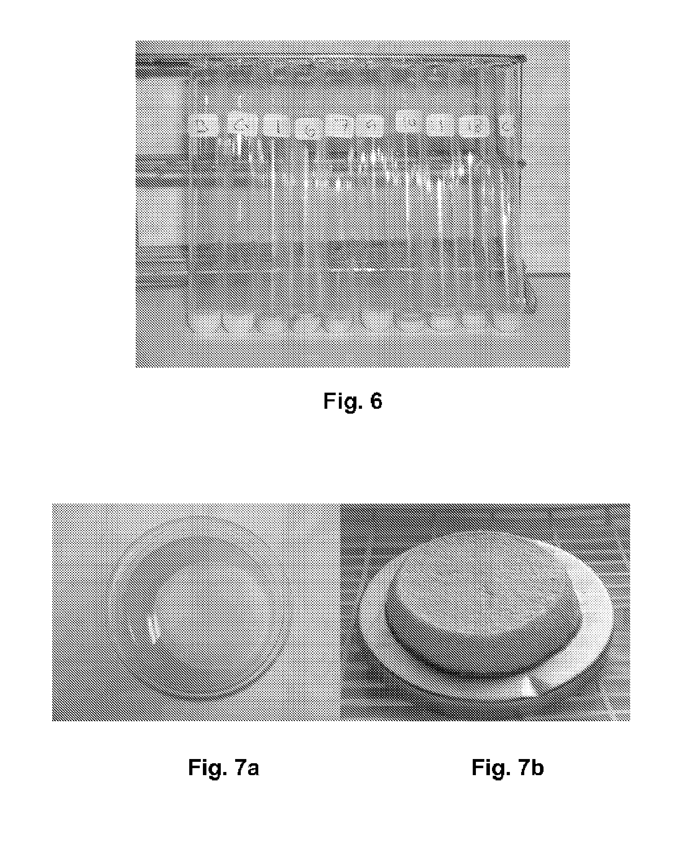

The sequences were cloned at the EcoRI cleavage site of the pGAPZαA vector. The constructions obtained were used to transform *E. coli* TOP1 0 cells. The *E. coli* clones carrying the vector with the sequence correctly orientated with respect to the pGAP promoter were detected by digestions of the plasmid with the *Kpn*I restriction enzyme. The vectors with the inserts in the appropriate orientation were linearised using the Bsp*H*I restriction enzyme and introduced into *Pichia pastoris* by means of electroporation using a Gene Pulser electroporator (BioRad) and following the manufacturer's instructions. The linear fragments integrated into the *Pichia pastoris* genome by recombination events. The yeast colonies carrying these constructions were selected on YPD plates supplemented with zeocine. The colonies obtained after selection were analysed by PCR using some oligonucleotides designed for regions flanking the point of insertion of the vector. Once the *Pichia pastoris* clones were obtained containing buffalo preprochymosin, prochymosin and chymosin integrated into their genome, they were cultivated in YPD without selective pressure (this is without zeocine) for 100 h at 30 ºC and 300 rpm. The cultures were centrifuged, obtaining supernatants with milk coagulant activity without the need for any prior activation. The coagulation assays (Fig. 6) were carried out on commercial powdered milk (Central Lechera Asturiana) dissolved in 13% potassium phosphate buffer 0.01 M, pH6. 200 µl of substrate were mixed with 200 µl enzyme sample. The negative control (C-) was the supernatant of the culture of a *Pichia pastoris* clone with the pGAPZαA vector without the insert, the target (B) was milk dissolved in buffer (as described above) and the positive control (C+) was a commercial rennet of animal origin.

As can be seen in Fig. 6, coagulation was produced with almost all cultures. In the negative control and in the target, no coagulation was produced, but coagulation was produced in the positive control, which validates the experiment.

Lastly, the supernatants of the different clones were used to make cheese masses using unhomogenised whole cows milk. These cheese masses were of high quality; good flavour, optimum firmness, good odour, etc. (Fig. 7).

To make cheese masses, the most active supernatant of the recombinant *Pichia* clones was used. The result was that the best construction for the production of active recombinant chymosin was that of prochymosin in the vector pGAPαA.

## Claims

1. Use of the rennet obtained from a host cell that contains an expression vector that comprises any of the sequences coding for the buffalo proteins selected from the group:
a. preprochymosin,
b. prochymosin or
c. chymosin for the coagulation of milk, where this rennet does not require prior activation.

2. Use of the rennet according to claim 1, where the host organism is *Pichia pastoris.*

3. Use of the rennet according to any of claims 1 and 2 for making cheese.

4. Use of the rennet according to claim 3 for making mozzarella cheese.


List of sequences
<110> University of Santiago de Compostela
<120> USE OF ACTIVE RECOMBINANT CHYMOSIN
<130> ES1596.8
<160> 4
<170> PatentIn version 3.4
<210> 1
<211> 24
<212> DNA
<213> Bubalus arnee bubalis infant
<220>
<221> misc_feature
<222> (1)..(24)
<223> Fragment of preprochymosin
<400> 1
atgaggtgtc tcgtggtgct actt 24
<210> 2
<211> 25
<212> DNA
<213> Bubalus arnee bubalis infant
<220>
<221> misc_feature
<222> (1)..(25)
<223> Procymosin
<400> 2
atggcagaaa tcaccagaat ccctc 25
<210> 3
<211> 23
<212> DNA
<213> Bubalus arnee bubalis infant
<220>

<221>  misc_feature
<222>  (1)..(23)
<223>  Chymosin
<400>  3
atgggtgagg tagcgagcgt gcc                                                    23
<210>  4
<211>  21
<212>  DNA
<213>  Bubalus arnee bubalis infant
<220>
<221>  misc_feature
<222>  (1)..(21)
<223>  Chymosin A
<400>  4 2
tcagatagcc ttcgccagcc c